# EUROPEAN PATENT APPLICATION

(11) **EP 2 044 912 A1**
(43) Date of publication of application: **08.04.2009**
(21) Application number: 08017316.4
(22) Date of filing: 01.10.2008
(51) Int. Cl.: A61F 9/02

(54) **Double lens**

(30) Priority: 03.10.2007 JP 2007259534
(71) Applicant: YAMAMOTO KOGAKU CO., LTD., Higashiosaka-shi Osaka (JP)
(72) Inventor: Kobayashi, Hirokazu, Higashiosaka-shi Osaka (JP); Yoshikawa, Keishi, Higashiosaka-shi Osaka (JP); Oka, Kouichiro, Higashiosaka-shi Osaka (JP)
(74) Representative: Koepe, Gerd L.

(57) **Abstract**

A double lens includes a front lens member (1), a back lens member (2) and a gasket (3). A back curve side of the front lens member and a front curve side of the back lens member are contraposed in parallel, and fixed together with the gasket, at least one of the back curve side of the front lens member and the front curve side of the back lens member has an antifogging film which is formed avoiding part of a gasket bonding area. This double lens provides goggles or a shield in which fogging is less likely to be generated, the gasket is less likely to be separated, and the front curve side of the front lens member is less likely to be damaged.

## Description

### TECHNICAL FIELD

The present invention relates to a double lens, which is suitable for a protective eyewear, particularly, sporting goggles or shield used in skiing, snowboarding, ice skating, cycling, and motocross or the like, and industrial goggles or shield used in constructing work, civil engineering work or the like.

In particular, the present invention relates to a double lens suitable for a protective eyewear such as goggles and a shield, which reliably functions to protect a user's eyes from being hit, rain, snow, dust or the like and is less likely to be fogged by a user's sweat or a change in the ambient temperature.

### BACKGROUND ART

In speed-involving sports such as skiing, snowboarding, ice skating, cycling, motocross and the like, goggles or shields which cover both eyes of a wearer are widely used in order to protect his eyes from an unexpected accident such as falling and crashing and in order to protect his eyes from a foreign substance such as rain and snow.

Furthermore, in a so-called dusty workplace for pulling down buildings, gathering soil and stones, crushing stones, digging tunnels, cement manufacturing, steel manufacturing, in mines or the like, in order to protect a wearer's eyes from a foreign substance, goggles and shields are widely used.

These goggles and shields are typically made of one fold lens structure, which is referred to a single lens.

Goggles and shields need to have a protective function from being hit and a blocking function against a foreign substance. Recently, these functions have been achieved up to a generally satisfactory level with appropriate device and choice of structure and/or materials to be used such as a resin.

The blocking function against a foreign substance relies on the eye-covering structure per se, and has little to do with the property of a resin used for a lens. On the other hand, the protective function from being hit has much to do with a bending rigidity and an anti-shock strength of a base material of a lens, and therefore the resin that is hard to be bent with resiliency and has toughness has been generally used.

However, when goggles or a shield is being put on a user, it is likely to be readily fogged due to his sweating and a change in the ambient temperature, which is unfavorable.

An antifogging function to goggles or a shield is provided by a method to apply an antifogging coating to an inner side of a lens, namely, a side facing a user's eyes of the lens. However, any antifogging agent has its limit in the amount of absorption of moisture, and water absorption exceeding the limitation generates water drops on the antifogging film, which further problematically causes a lens to be fogged.

Further, the side facing a user's eyes of goggles or a shield and the body of the user define a kind of a sealed space, and this space will be filled with moisture such as sweat continuously issued from the user's skin. Therefore wearing goggles or a shield continuously long time inevitably causes fogging to be generated sooner or later. Thus it has been difficult to impart a semi-permanent anti-fogging property to goggles and shields.

In view of the foregoing limitation in the antifogging function, there has been a suggestion of a dual lens structure, or a double lens, in which two lens members are disposed generally in parallel and fixed with a gasket provided on the peripheral parts of the lenses.

Out of the two lens members forming the double lens, a lens member facing outward, or a front lens member, has been in general made of a polycarbonate resin in order to provide an anti-shock function.

On the other hand, an inner lens member facing a user's eyes, or a back lens member, has been preferably made of an acylcellulose resin having a hygroscopic nature and an antifogging property, in particular, propylcellulose.

The double lens has a space defined by the two lens members and the gasket as a temperature buffering zone, so that, even if the goggles are brought into contact with cold outside air, cooling of the inner side of the goggles is relieved.

As a result, filling vapor near a user's body is less likely to be condensed into dew on the back lens member. Even if condensed into dew, it is absorbed in the acylcellulose resin and fogging on the side facing a user's eyes of the double lens in goggles or a shield is less likely to be generated compared with the fogging on the single lens.

However, an acylcellulose resin generally has a high moisture permeability, so that filling vapor near a user's body easily penetrates into the interior part of the double lens defined by the front lens member, the back lens member and the gasket. If a user with the goggles came out from a warm room to a cold environment below -5°C such as a ski run, he had a problem that an inside of a front lens member, namely, a back curve side of the front lens member became fogged.

To solve the above problem, providing a coating with an antifogging property to a back curve side of the front lens member was suggested in Japanese Patent Application, Unexamined Patent Publication No. 2002-505157.

However, from a viewpoint of a manufacturing technology, it is not easy to provide an antifogging film only to the back curve side of the front lens member made of a polycarbonate resin by injection molding.

For example, a simple dipping method provides antifogging coasting not only to the back curve side but also to the front curve side at the same time. Since the antifogging film generally has a low hardness, it is prone to be damaged when the surface of goggles or the shield is wiped, and repetitive wiping and damaging eventually cause the lens to be like a frosted glass and deteriorate its visibility.

In addition, even if the antifogging coating is applied only to the back curve side of the front lens member using a complicated technology, an antifogging film generally has its own low strength and a low adhesion to a base material, and therefore part fitted with a gasket is easily peeled off and thereby resulting in a problem that a durable double lens cannot be produced.

For solving the problem of low adhesion, there has been an idea that part to be fitted with a gasket is not treated with an antifogging film, however, any tangible technique has not been suggested.

### SUMMARY OF THE INVENTION

In order to solve the above-described problems, following measures are taken.

The double lens includes is a front lens member, a back lens member and a gasket. A back curve side of the front lane member and a front curve side of the back lens are contraposed in parallel, and fixed together with the gasket to form the double lens. At least one of the back curve side of the front lens member and the front curve side of the back lens member has an antifogging film which is formed avoiding part of a gasket bonding area. The antifogging film is formed after the gasket is fixed on the side to be provided with the antifogging film or a masking member with an equivalent shape is applied on the side.

Further, the double lens may be prepared with the antifogging film formed by a printing method avoiding the corresponding shape of the gasket.

Further, the two lens members may be fixed with the gasket before having the antifogging film and an antifogging solution may inserted inside of the double lens through an air hole in the lens or the gasket to form the antifogging film.

The present invention provides the double lens suitable for goggles or a shield which has an excellent function to protect a user's eyes from hitting, rain, snow, dust and the like, is less likely to be fogged in spite of the user's sweating or the change in ambient temperature, and has durability, so that preferably used in playing sports such as skiing, snowboarding, ice skating, cycling and motocross and in industrial sites such as in construction and civil engineering.

### BRIEF DESCRIPTION OF THE DRAWINGS

Fig. 1 is an external perspective view of ski goggles, which is an optical eyewear structure carrying out a double lens according to the present invention.
Fig. 2 is a sectional view of the ski goggles in Fig. 1.
Fig. 3 is an explanatory view seen from the side of a back lens member of the double lens according to the present invention, which is carried out in the ski goggles in Fig. 1.
Fig. 4 is a sectional view of ski goggles according to another embodiment.

### DESCRIPTION OF SPECIFIC EMBODIMENTS

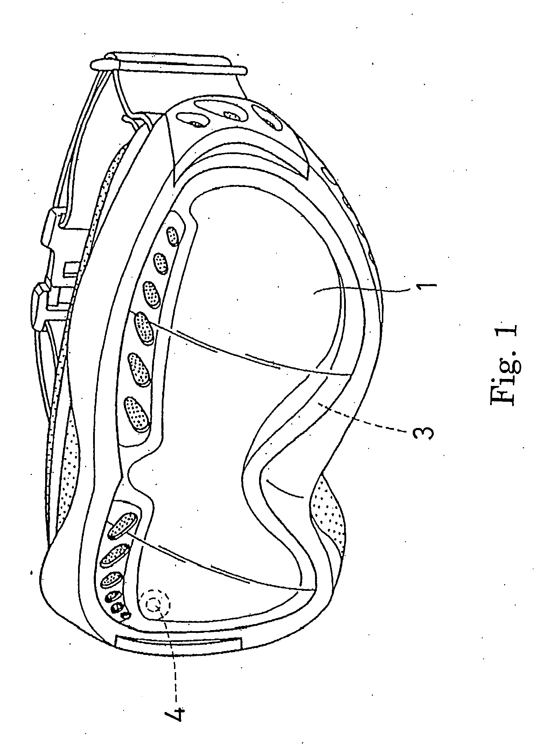

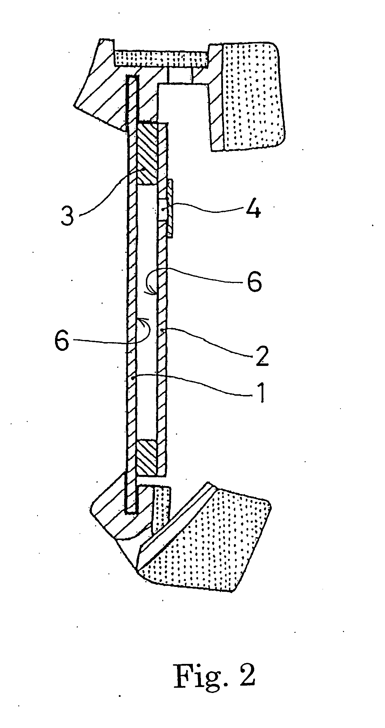

The double lens according to the present invention includes two resin lens members; for example, as shown in Figs. 1 to 3, a front lens member 1 and a back lens member 2 which are to be used for ski goggles of an optical eyewear structure. The two lens members are placed in contraposition in parallel and fixed together by a gasket 3. As a result, the double lens is provided with a space defined by the front lens member 1, the back lens member 2 and the gasket 3.

Resins to be used for the front lens member 1 of the present invention preferably include a vinyl chloride resin, an acrylic resin, a polystyrene resin, an ethylene-vinyl acetate resin, a vinylidene chloride resin and an acetyl cellulose resin, which have a highly transparent resin. In particular, a polycarbonate resin, a polyurethane resin, a polyester resin and a polyamide resin which have transparency, hardness, bending strength and toughness are more preferable.

The polycarbonate resin includes an aromatic polycarbonate resin such as a bisphenol A type, an alicyclic polycarbonate resin, and a polymer alloy of a polycarbonate resin and a polyester resin.

Particularly, in consideration of hardness, strength and toughness, a polycarbonate resin containing an aromatic ring such as a bisphenol-A and a polymer alloy of this polycarbonate resin and a polyester resin are preferable.

As the polyurethane resin, a highly transparent polyurethane resin which is less likely to be crystallized is preferable among polyurethane resins in which an isocyanate component mainly containing an aromatic isocyanate and an alicyclic isocyanate.

As the polyester resin, a highly transparent polyester resin is preferable among polyester resins in which a dicarboxylic acid mainly containing an aromatic dicarboxylic acid such as a terepthalic acid.

The polyamid resin preferably includes an amorphous and highly transparent polyamid resin which contains an alicylic or an aliphatic dicarboxylic acid and an alicylic or an aliphatic diamine in a molecule.

Among the polycarbonate resin, the polyurethane resin, the polyester resin and the polyamid resin as stated above, the polycarbonate resin which is excellent in hardness, bending strength, and toughness is preferably used.

Particularly, a polycarbonate resin containing an aromatic ring such as a bisphenol-A or a polymer alloy of this polycarbonate resin and a polyester resin, of which viscosity average monocular amount in the polycarbonate resin is not less than 15,000, preferably, not less than 18,000 is strongly recommended because both are excellent in bending strength and toughness and have an excellent function in preventing a wearer of the goggles or the shield with the double lens of the present invention from a shock.

The front lens member 1 can be generally produced by an injection molding method, an extruding molding method, a press molding method or a cast molding method.

In a case of making a front lens member 1 according to the present invention of polycarbonate resin, polyurethane resin, polyester resin or polyamid resin, it is preferable to form the front lens member 1 into a cylindrical shape, an aspheric shape or a spherical shape respectively by using a mold of a cylindrical shape, an aspheric shape or a spherical shape.

Particularly, an aspheric shape or a spherical shape may be preferable from an aspect of a design or for omnidirectional correction of a refracting power.

The aspheric shape herein denotes the shape that deviates somewhat from a true spherical surface due to a requirement for a design to aim for an attractive appearance or an optical requirement to reduce distortion of vision as much as possible in any region of the lens when a user wears goggles or a shield.

In addition, the spherical shape herein denotes a true spherical surface in which a front curve and a back curve are respectively parts of an arc in any cross section of the lens.

Further, the front curve herein denotes is a curve on the side facing an object to be seen of the lens, and the back curve is a curve on the side facing an user's eye of the lens.

A lens designed to have a uniform thickness is called a plano lens. Particularly, in a case of a horizontally long plano lens used for like single-lens goggles or a single-lens shield, an acuter angle of incidence and an increase in length of a path within a base material of the lens cannot be avoided nearer to the right and left ends than the center part of the lens. As a result, a larger refracting power is generated and a distortion of vision becomes larger nearer to the ends of the lens. In other words, a wearer of the goggles or the shield with the horizontally long lens faces a problem that his view is easily distorted when seeing an object through the right and left end portions of the lens.

Such a refracting power of the lens having a uniform thickness can be corrected in an injection molding method by reducing the thickness toward the ends of the lens.

In other words, based on an optical theory to correct a refracting power, by using an aspheric mold of which thickness for a lens is designed so as to become thinner from the center of the lens toward the ends of the lens, an aspheric lens of which distortion of vision is corrected is produced.

In addition, in the same way, by using a mold in which a back curve degree is adjusted relative to a front curve degree, the thickness of the lens is made thinner toward the ends so as to obtain a spherical lens in which distortion of vision is corrected.

Further, the curve degree here means (1.523 - 1)/R, where 1.523 is a refraction index of a glass and R(m) is a curvature radius.

Further, the theory to correct the refracting power holds true in the case of a cylindrical shape lens.

Such a correction of a refracting power is advantageous especially in a case of an injection molding method and a cast molding method using a mold.

In a case of the front lens member 1 of which refracting power is not corrected, the thickness of the lens of 0.3 to 3 mm is preferable, and particularly, the range of 0.4 to 2.6 mm is recommended.

If the thickness is lower than 0.3 mm, the lens is easily curved or bent and a physical strength thereof is weakened, and if the thickness is higher than 3 mm, the refraction power at the ends of the lens is likely to increase, thereby both are not preferable.

In a case of correcting the refracting power, the center thickness of the lens is preferably 0.8 to 4 mm, particularly, the range of 1 to 3.6 mm is recommended.

If the center thickness is lower than 0.8 mm, the physical strength is easily weakened at the ends of the lens, while if the center thickness is larger than 4 mm, the lens per se likely increases weight, and both are not preferable.

In addition, a resin sheet can be used for the front lens member 1. The method used in this case is as follows: a resin sheet of thickness of 0.3 to 3 mm, more preferably, 0.4 to 2.6 mm is made by injection-molding through a slit into a sheet-like shape, press-molding or cast-molding, and this resin sheet is reshaped into an aspheric shape, or a spherical shape or a cylindrical shape.

If the sheet has a thickness lower than 0.3 mm, it is easily curved or bent when formed into a lens for goggles or a shield, while if it has a thickness larger than 3 mm, an obtained lens increases a refracting power at the ends, and both are not preferable.

A method of reshaping a resin sheet into an aspheric shape, a spherical shape or a cylindrical shape is not particularly limitative, however, a popular reshaping method is as follows: a resin sheet prepared as above is cut into an appropriate size, this cut resin sheet is set in a mold for reshaping and heat-reshaped into a given shape by means of hot air, a hot trowel or the like.

When a front lens member 1 is formed by injection-molding or cast-molding, a deflection plate or a photochromatic resin plate which is reshaped into the same aspheric, spherical or cylindrical shape as the front lens member 1 can be set within the mold in advance, and insert-molded on the surface of the lens.

In addition, the added value of the front lens member 1 can be enhanced by applying hard-coating, antireflection-finishing and water-repellent finishing to the surface thereof.

In addition, due to a vacuum deposition technique or the like, a metallic, or a half-mirror, or a special color phased super thin film can be also provided thereon.

In addition, the front lens member 1 can be colored by applying a dye or a colorant into a base material resin in advance, or by staining a prepared lens member or a resin sheet with a dye.

The resin to be used for the front lens member 1 can be generally used for the resin to be used for the back lens member 2 of the present invention.

Particularly, a polycarbonate resin, a polyurethane resin, a polyester resin and a polyamide resin having a transparency, a hardness, a bending strength and a toughness are preferable.

In addition, an acylcellulose resin having a transparency, an antifogging property and a certain physical strength is also preferably used.

The polycarbonate resin includes an aromatic polycarbonate resin containing an aromatic ring such as a bisphenol A type, an alicyclic polycarbonate resin and a polymer alloy of a polycarbonate resin and a polyester resin.

Particularly, in consideration of hardness, strength and toughness, a polycarbonate resin containing an aromatic ring such as a bisphenol-A and a polymer alloy of this polycarbonate resin and a polyester resin are preferable.

In addition, as the polyurethane resin, a highly transparent polyurethane resin less likely to be crystallized is preferable among polyurethane resins in which an isocyanate component mainly containing an aromatic isocyanate and an alicyclic isocyanate.

In addition, as the polyester resin, a highly transparent polyester resin is preferable among polyester resins in which a dicarboxylic acid component mainly containing an aromatic dicarboxylic acid such as a terepthalic acid.

In addition, a preferable resin for the polyamid resin is an amorphous and highly transparent polyamid resin which contains an alicylic or an aliphatic dicarboxylic acid and an alicylic or an aliphatic diamine in a molecule.

In addition, the acylcellulose resin preferably includes an alkylcarboxylic acid (mono, di, tri) ester of cellulose such as an acetylcellulose and a propylcellulose, a mixture thereof, or an ester mixture. Particularly, a propylcellulose resin excellent in an antifogging property and a molding capability is more preferable.

The acylcellulose resin may be added with a plasticizing agent in order to enhance a plastic property.

In addition, it is preferable that a surface of a lens of the acylcellulose resin is treated with an alkali in order to enhance an antifogging property.

Among the polycarbonate resin, the polyurethane resin, the polyester resin, the polyamid resin and the acylcellulose resin stated above, the polycarbonate resin is particularly and preferably used in view of hardness, bending strength and toughness of the resin, while the propylcellulose resin is particularly and preferably used in view of an antifogging property of the resin.

A particularly recommendable polycarbonate resin is a polycarbonate resin containing an aromatic ring such as a bisphenol A type, or a polymer alloy of this polycarbonate resin and a polyester resin in which a viscosity average monocular amount of the polycarbonate resin is not less than 15,000, preferably, not less than 18,000 because it is excellent in bending strength and toughness and reliably functions to prevent a wearer of the goggles and the shield from a shock.

In addition, the preferable propylcellulose resin is a (di or tri) propylcellulose, a mixture of a (mono, di, tri) propylcellulose, or a mixture in which a propylcellulose is mainly present and mixed with an acetylcellulose or other acylcellulose.

The back lens member 2 according to the present invention can be produced manufactured by an injection molding method, a press molding method, a cast molding method or an extruding molding method like the front lens member 1.

In a case of using a mold like in an injection molding method and a cast molding method, a refraction power corrected lens having thinner ends as well as a lens having a uniformed thickness can be produced.

Especially, the back lens member 2 preferably used in the present invention is the back lens member 2 having a uniform thickness and made of a resin sheet in view of easiness and cost of production.

It is recommended that the back lens member 2 has a thickness of 0.3 to 3 mm, or preferably 0.4 to 2.6 mm.

If a lens has a thickness lower than 0.3 mm, it is likely to be curved or bent.

In addition, if a lens has a thickness exceeding 3 mm, like the front lens member 1, a distortion of vision at the ends of the lens due to a refracting power is liable to increases.

It is preferable that the back lens member 2 according to the present invention has an aspheric shape, a spherical shape or a cylindrical shape which is substantially similar to the shape of the front lens member 1. Forming the back lens member 2 into the shape substantially similar to that of the front lens member 1 allows to dispose these lens members opposing each other in parallel, and thereby resulting in a generally uniformed thickness and an attractive appearance of the double lens.

A method of giving the back lens member 2 the shape substantively similar to that of the front lens member 1, in other words, a method of forming the back lens member 2 in an aspheric shape, a spherical shape or a cylindrical shape is not limitative, however, a typical method is a method of using a mold similar in shape to that of the front lens member 1 or a method in which a resin sheet is cut into an appropriate size, this cut sheet is set into a mold for reshaping and heat-reshaped into a shape similar to that of the front lens member 1 by means of hot air, a hot trowel or the like.

Alternative methods include a method in which the front and back lens members 1, 2 of resin sheet and a gasket 3 are fixed together while being bent into a cylindrical shape and a method in which the front and back lens members 1, 2 of resin sheet are fixed together with a gasket 3 without reshaping in advance into a double lens and the resultant double lens is bent and fitted into a lens receiving hole of a goggle frame or a shield frame curved in a cylindrical shape.

Especially, in a case that the back lens member 2 is made of a synthetic resin such as the polycarbonate resin, the polyurethane resin, the polyester resin and the polyamide resin, it is preferable that the back curve side of the back lens member 2 is provided with an antifogging treatment since water issued from a wearer's body is liable to generate condensation on this side.

For this purpose, it is recommended that an antifogging solution is applied so as to form an antifogging film. In a case of the back lens member 2 of the acylcellulose resin, as described above, it is recommended that an antifogging property is provided on the surface of the lens member by a hydrolysis treatment due to alkali treatment.

In addition, the back lens member 2 may be provided with a functional film such as a hard coat and a deposited film like the front lens member 1.

Water issued from a user's body and water in the air may pass through the front and the back lens members 1 and 2 into the inner space defined by the front and back lens members 1 and 2 and the gasket 3. And in a cold environment such as in a ski run, the double lens is liable to have condensation thereon.

In the present invention, in order to prevent condensation, an antifogging film 6 is formed on at least one of the back curve side of the front lens member 1 and the front curve side of the back lens member 2.

Particularly, the back curve side of the front lens member to be directly exposed to an outside temperature is liable to have condensation and therefore preferable provided with an antifogging film 6.

However, if antifogging solution is applied to a lens member by a dipping method or a spin coat method, the lens member is entirely coated and the antifogging film is formed on the entire surface of the lens member. Therefore, when two such lenses are fixed together by a gasket 3, they are fixed with a bonding agent or an adhesive agent through the antifogging film.

The antifogging film in general has a low adhesion with a base material of the lens member. In addition, the strength of the film upon absorption of moisture is particularly weak. Therefore, even if the two lens members are fixed to the gasket 3 with an adhesive or bonding agent, separation is liable to arise in an interface between the lens members and the antifogging film or within the antifogging film. This leads to a problem that a durable double lens, namely, durable goggles and a durable shield cannot be produced.

In order to prevent such separation in the double lens, in the present invention, an antifogging film 6 is formed on the lens member(s) avoiding the part where the gasket 3 is to be adhered so as to produce a double lens having no antifogging film on the part where the gasket 3 is to be attached.

For the above purpose, there is a method in which an antifogging film is applied after a gasket 3 is fixed on a surface which is later to be applied with the antifogging film or after part of the surface is covered with a masking body having an equivalent shape. Herein, the masking body means a masking sheet or a packing for masking.

In other words, in this method, after the gasket 3 is fixed or a masking having an equivalent shape is attached, an antifogging solution is applied by a dipping method, a spin coating method, a printing method or a vapor depositing method, or an antifogging film is formed by vapor deposit-forming, with no antifogging film on the part where the gasket 3 is to be fixed.

In addition, there is another method using no masking body, in which the surface other than the part of an equivalent shape of the gasket 3 is applied with the antifogging solution by a printing method to have no antifogging film only on the part where the gasket 3 is to be fixed.

The printing method includes a stamp system such as a so-called pad printing, a screen printing method and an inject printing method.

In addition, there is another method employed after a double lens is assembled, in which an antifogging solution is injected into the interior of the double lens through a though hole to be discuss later (an air pressure adjusting hole of a lens member or a pore or an air pressure adjusting hole of the gasket 3) and rinsed therein to form a coating thereof, and the remainder is discharged through the hole, so that no antifogging film is obtained only on the part where the gasket 3 is to be fixed.

Further, in the case that the back lens member 2 is made of the acylcellulose resin, it may be treated with an alkali in advance to obtain an antifogging property, which necessitates to form no antifogging film. Further, the obtained antifogging property doesn't prevent a fixing property of the gasket 3. The foregoing is particularly recommendable.

The antifogging solution to be used for the present invention includes an surfactant series, a hydrophilic polymer origin such as a polyvinyl alcohol, a polyvinyl pyrolidone, a sodium polyachrylate, an alginate sodium, agar and gelatin, a heat or activated light curing series which mainly contains a hydrophilic monomer, and a mixture thereof.

These compounds are commonly dissolved into a solvent such as water and alcohol if necessary, added with a leveling agent or a polymerization start agent if necessary, dried or cured after coating and thus resulting in an antifogging film.

In addition, in a case of vapor deposition, a silicon oxide film such as SiO₂ is typical, and a vapor-deposited film may be further provided with a hydrophilic compound such as an surfactant.

In the present invention, the double lens may be produced by adhering or bonding the front and back lens members 1 and 2 in a fashion that they are contraposed in parallel having the gasket 3 therebetween, although this depends on the method of forming the antifogging film avoiding the part to which the gasket 3 is to be fixed.

A polyurethane resin, a synthetic rubber, an elastomer or the like is suitable for the base material resin of the gasket 3. Particularly, a resin or a porous resin having a sufficient elasticity to be deformable when pushed by a user's finger, then restorable of itself to a original form, and having a fracture elongation not less than 40% is preferable.

Particularly, a porous gasket of which elasticity and elongation is readily adjustable to a preferable degree and of which weight can be reduced is recommended.

The porous gasket preferably has a closed-cellular structure in order to prevent the wet of sweat, snow and/or rain from easy penetration into the interior of the double lens.

The gasket 3 is preferably colored by a colorant or a dye for an aesthetic purpose of the goggles. And there is a trend toward preferring an achromatic color phase such as black or white.

As a form of the gasket 3 may be a string. A string-shaped gasket is made by shaping the above-described base material resin into a string having an equivalent diameter of 1 to 8 mm, more preferably, 3 to 6 mm.

A gasket with an equivalent diameter smaller than 1mm cannot maintain a sufficient interval between the front and the back lens members 1 and 2, which may cause the two lens members to come into contact with each other and lose adhesion. On the other hand, a gasket with a diameter over 8mm is liable to spoil the appearance of the goggles.

The method to make a double lens using a string-shaped gasket is as follows: a string-shaped gasket is applied with an adhesive or a bonding agent, then put on the part with no an antifogging film thereon on the back curve side of the front lens member 1 or on the front curve side of the back lens member 2, then after or before forming a antifogging film, the front lens member 1 is placed on the back lens member in a manner they stand in contraposition and in parallel, and then the two lens members are adhered and fixed together with addition of heat or pressure if necessary to produce a double lens.

In addition, the gasket 3 may be a sheet-like gasket, and a double lens may be produced with the sheet-like gasket in the same way and process as to the string-shaped gasket.

A common method for this purpose is as follows: the sheet made of the above-described base material resin having a thickness of 0.5 to 7 mm, preferably, 1 to 5 mm is punched or cut out into a shape with a width of about 2 to 10 mm, preferably, 3 to 8 mm in accordance with the shape of a bonding area of the double lens, and the resultant sheet is attached to the front and the back lens members 1 and 2 with an adhesive or a bonding agent.

A gasket with a thickness smaller than 0.5mm cannot maintain a sufficient interval between the front and the back lens members 1 and 2, which may cause the two lens members readily to come into contact with each other. On the other hand, a gasket with a thickness over 8mm tends to mar the looks of the goggles.

A gasket with a width smaller than 2 mm is liable to lose a sufficient adhesion of an adhesive and a bonding agent, while a gasket with a width over 10 mm tends to mar the appearance of the goggles.

Further, the gasket 3 does not necessarily have a uniform width and the width thereof may appropriately vary according to a design and/or a structural necessity. The word "about", for example, in "a width of about 2 to 10 mm" shown above, implies such a reason.

The most preferable gasket 3 of the present invention is a sheet-like gasket from the viewpoint of easiness of production and workability. In view of workability and reliability of bonding, the most preferable method is as follows: an adhesive or a bonding agent is applied to the both faces of a sheet for a gasket 3, the gasket sheet is further covered with a release sheet and punched or cut out into a shape according to the shape of the bonding area of a double lens so as to make a gasket 3 in an annular shape or the like, then after the release sheet is removed, the gasket is placed on the part with no antifogging film on the back curve side of the front lens member 1 or the front curve side of the back lens member 2, then after or before the forming of an antifogging film, the front lens member 1 and the back lens member 2 are attached to each other in a manner that they are contraposed in parallel, and then they are adhered or fixed together by heat or pressure if necessary to produce a double lens.

The adhesive of the gasket 3 includes an acrylic type and a synthetic rubber type. The bonding agent thereof includes a vinyl acetate type, a cyanoacrylate type, a polyurethane type and an elastomer type. From the viewpoint of workability and reliability of bonding, the adhesive is more preferable.

As described above, the front lens member 1, the back lens member 2 and the gasket 3 form one double lens having a space defined by the front lens member 1, the back lens member 2 and the gasket 3.

It is preferable that this space is a sealed space so as to prevent invasion of rain and sweat during playing sports or working.

When skiing and snowboarding, a user takes a lift or a gondola many time a day and plays such sports in different altitudes. If a double lens has a sealed space, it becomes inflated or deflated depending on the change in air pressure. Consequently, when the bonding face of the gasket 3 cannot withstand the change in air pressure, it is liable to separate from the lens.

In order to solve such a problem, it is recommended in the present invention that the double lens has an air pressure adjusting hole 4 to the space defined by the front lens member 1, the back lens member 2 and the gasket 3.

In the method of forming an air pressure adjusting hole, the hole may be formed on in the lens or the gasket 3.

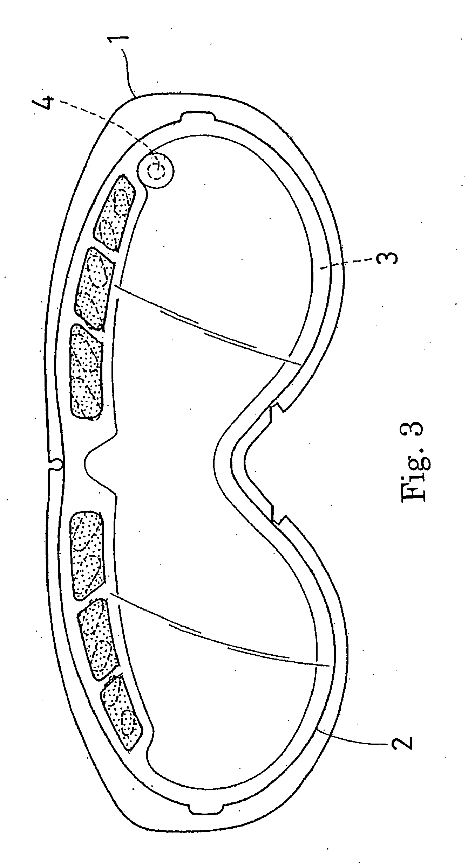

In the case of forming an air pressure adjusting hole in the lens, a hole, a slit or the like is formed in the lens. Especially, a method of forming the air pressure adjusting hole in the back lens member 2 as shown in Fig. 3 is preferable, which is less likely to spoil an appearance in use.

In this case, at least one air pressure adjusting hole is formed in the back lens member 2. In light of an aesthetic view, one air pressure adjusting hole 4 is desirable.

The shape of the air pressure adjusting hole 4 is not particularly limitative, however, a circle and an oval shape are preferable since these shapes is less likely to deteriorate the physical strength of the lens, and also preferable for the aesthetic view.

In addition, for the size of the air pressure adjusting hole 4, an equivalent diameter of 0.5 to 3 mm, preferably, 0.6 to 2.5 mm is recommended. One air pressure adjusting hole with the equivalent diameter lower than 0.5 mm is liable to cause a malfunction in air pressure adjustment. The equivalent diameter higher than 3 mm tends to spoil the appearance of the goggles or the like.

The present air pressure adjusting hole 4 is preferably provided with some waterproof treatment. As the waterproof treatment, a recommendable method is to past a sheet over the hole, the sheet having a function to pass vapor but lesser water.

Such a sheet includes a fluorinated minute porous sheet such as Gore-Tex manufactured by Japan Gore-Tex Inc., a polyurethane sheet, an open-cellular minute porous sheet with or without water repellent treatment, a waterproof cloth having a water repellent agent sprayed or applied, and a fabric product made by weaving or knitting a fabric with water repellent treatment.

Such sheet-like substance is pasted over the air pressure adjusting hole by an adhesive or a bonding agent.

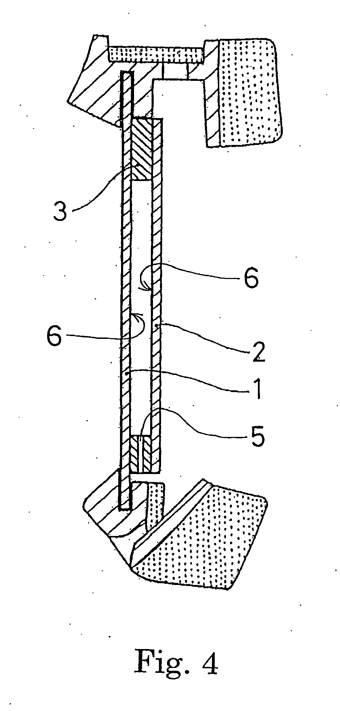

In addition, in the case of forming an air pressure adjusting hole in the gasket 3, as shown in Fig. 4, at least one air pressure adjusting hole 5 is formed in the gasket 3.

As such an air pressure adjusting hole 5, a small cut line through the gasket 3 is recommended. In a case of a porous gasket, the gasket 3 may have a open-cellular structure.

Both the case providing a small cut line through the gasket 3 and the case using a porous gasket, in order to prevent invasion of rain from outside and sweat, applying a sheet having a function to pass vapor but not lesser water over the hole of the gasket is preferable for the former case, while waterproof treatment on the surface of the gasket by spraying or applying the water repellent agent thereto is preferable for the latter case.

The double lens according to the present invention is generally an injection-molded structure, and if used for goggles, this double lens is mounted on a goggle frame having a hole to fitly receive the double lens and a cushiony put-on-face member which gives a gentle and comfortable fitting to a user's face, but not limitative thereto.

In addition, it is preferable that the goggle frame has an air hole to outside air so that air within the goggles can be ventilated.

As a resin to be used for the goggle frame, a resin having a modest softness is recommended in view of workability in fitting the double lens and comfortability to a user's face.

The double lens is fitly received in a fitting hole of the goggle frame, and in general the double lens is fixed in a groove formed in a constituent wall of this fitting hole. In some cases, the double lens may be bonded by a adhesive or a bonding agent to the goggle frame.

The air hole to outside air to be formed in the goggle frame plays the role to ventilate interior air of the goggles in use so as to prevent moisture issued from a user's body from filling the goggles. In other words, the air hole plays the role to prevent the back curve side of the back lens member 2 from fogging and vapor from invading in the double lens through the back lens member 2.

The air hole is generally formed on the upper side, the side faces, or the lower side of the goggle frame.

The size and the number of the hole are not particularly limited, however, in order to heighten a ventilation property, an area thereof not less than 1 cm² is preferable.

In addition, if the air hole is an open hole, rain, snow and/or sweat may invade and thus it is normally preferable that some waterproof treatment is provided to the air hole. A recommendable waterproof treatment is to paste a sheet having a function to pass vapor but not water over the hole.

Such a sheet includes a fluorinated minute porous sheet such as Gore-Tex manufactured by Japan Gore-Tex Inc., a polyurethane sheet, an open-cellular minute porous sheet with or without water repellent treatment, a porous body, a mesh body, a waterproof cloth sprayed or applied with a water repellent agent, and a fabric product made by weaving or knitting a fabric with water repellent treatment.

Such sheet-like substance, the porous body or the mesh body is pasted over the air hole of the goggle frame by an adhesive or a bonding agent.

In addition, in order to enhance a fitting comfortability to a user's face, the goggle frame is normally designed into a generally cylinder shape in accordance with a contour of a user's face.

In addition to this, a put-on-face member is in general provided on the part contacting the user's face so as to improve a fitting comfortability to a user's face and a cushiony property, and prevent discomfortability such as pain and itch on a user's face.

The on-face member is a kind of a cushion material, which is made of a resin softer than that of the goggle frame. In view of a comfortable touch, a porous resin is preferable. It may be either open-cellular or closed cellular, however, it is preferable to be open-cellular with a ventilation property.

The goggle frame and the on-face member are in general made of different resins, and they are bonded together by an adhesive or an bonding agent or they are fixed together by a fastening face member.

The double lens according to the present invention may be used as a shield or as an incorporated part of the shield.

On the goggle frame or the shield, a belt or a clip is provided so as to put it on a user's head or mount it to a helmet.

### [First Embodiment]

### (Production of goggles)

### (1) Preparation of a front lens member 1:

Using a polycarbonate resin of a bisphenol A type of which viscosity average molecular amount is about 22,000, a front lens member 1 with a front curve 6C, a back curve 6.1C and a center thickness 1.8 mm was injection-molded.

The front curve side of the front lens member 1 was applied with a mirror finish by a vacuum depositing method.

### (2) Formation of an antifogging film on the back curve side of the front lens member 1:

The front lens member 1 in which a masking sheet was adhered to a front curve side and a gasket-bonding part on a back curve side was dipped into a solution for forming an antifogging film mainly containing a polyvinyl alcohol and a surfactant. An antifogging solution was applied to the front curve side and the back curve side avoiding the gasket-bonding part, and then the front lens member was dried. And an antifogging film 6 was formed on the back curve side avoiding the gasket-bonding part.

After that, the masking sheet was peeled off.

### (3) Preparation of a back lens member 2:

A propylcellulose sheet mainly containing a tri-propylcellulose of a thickness of 0.8 mm was dipped into an alkaline solution to have an antifogging property on the sheet surface thereof. Then, this propylcellulose sheet was punched out into a size approximately same as the front lens member 1.

On the position near the gasket 3 to be provided ton he back lens member 2 as much as possible, a circular hole of a diameter of 1 mm was punched out to form an air pressure adjusting hole 4.

Subsequently, the resultant propylcellulose sheet was set in a mold, and hot-reshaped into a shape similar to the back curve of the front lens member 1 to form a back lens member 2.

### (4) Preparation of a gasket 3:

An adhesive was applied on the both faces of the closed-cellular sheet made of a chloroprene of a thickness of 3 mm, the resultant sheet was further covered with a release sheet and then punched out into a fixing shape of the double lens to form a ring-like gasket of a width of 5 mm.

### (5) Preparation of a double lens:

The release sheet on one side of the ring-like gasket 3 was peeled off, and the gasket was fixed to the front curve side of the back lens member 2.

The other release sheet on the other side was peeled off. The front lens member 1 having the antifogging film but other than on the gasket-bonding part was contraposed in parallel with respect to the back lens member and pressed thereto to fix the members together.

### (6) Waterproof of an air adjusting hole 4 of the double lens:

A fluorinated porous sheet of a diameter of 3 mm having an adhesive ("Gore-Tex" manufactured by Japan Gore-Tex Inc.) was pasted over the air adjusting hole 4 of a diameter of 1 mm formed on the back lens member 2 to make the hole waterproof.

### (7) Preparation of a goggle frame:

The mold provided with a hole to fitly receive the double lens part and four air holes of 1 cm² on the upper side of the goggle frame, and designed to be a cylindrical shape so as to easily fit with a user's face was used. With this mold, a polyurethane resin was injection-molded so as to prepare the goggle frame.

### (8) Waterproof of the air hole of the goggle frame:

An open-cellular polyurethane sheet of a thickness of 2 mm was pasted over each air hole with an adhesive.

### (9) Attachment of the goggle frame to a on-face member and finishing of the goggle frame:

An open-cellular polyurethane sheet of a thickness of 15 mm was cutting out into a shape with a width of 15 mm in accordance with a shape of a face contacting part of the goggle frame to form a ring-shaped on-face member. It was pasted on a periphery part of the goggle frame by an adhesive to finish the goggle frame.

### (10) Attachment of the double lens to the goggle frame:

The double lens was inserted in a receiving hole of the goggle frame and fitted in a groove around the receiving hole so as to fix the double lens to the frame.

### (11) Preparation of a fitting belt to a user's head and finishing of the goggles:

A fitting belt to a user's head was made with a belt of elastic string with rubber sting wounded by synthetic fiber and a clip. The belt was attached to the goggle frame to form goggles.

### (Antifogging Test)

Putting the above-described goggles to and on his face in at an ambient temperature, a wearer entered an environmental test room at a temperature of -5°C. Even after one hour had passed, no fogging was generated on any region of the double lens.

In addition, even if the double lens was twisted, no separation was found in any fixing part between the front lens member 1, the back lens member 2 and the gasket 3.

### [Second Embodiment]

### (Production of goggles)

### (1) Preparation of a front lens member 1:

Using a transparent nylon resin ("Dyamyd" ZC7500, manufactured by Daicel-Degussa LTD.), a front lens member 1 of a front curve 6C, a back curve 6.1C, and a center thickness 1.8 mm was injection-molded.

The front curve side of front lens member 1 was applied with a mirror finish by a vacuum depositing method.

### (2) Preparation of a back lens member 2:

A propylcellulose sheet having a thickness of 0.8 mm was dipped into an alkaline solution and an antifogging property was given to the sheet surface thereof. Then, this propylcellulose sheet was punched out into a size approximately same as the front lens member 1.

On the position near the gasket 3 to be provided on the back lens member 2 as much as possible, a circular hole of a diameter of 2 mm was punched out to form an air pressure adjusting hole 4.

Subsequently, the resultant propylcellulose sheet was set in a mold, and hot-reshaped into a shape similar to the back curve of the front lens member 1 to form a back lens member 2.

### (3) Preparation of a gasket 3:

An adhesive was applied on the both faces of the closed-cellular sheet made of a chloroprene of a thickness of 3 mm, the resultant sheet was further covered with a release sheet and then punched out into a fixing shape of the double lens to form a ring-like gasket 3 of a width of 5 mm.

### (4) Preparation of a double lens:

The release sheet on one side of the ring-like gasket was peeled off, and the gasket 3 is fixed to the front curve side of the back lens member 2. The other release sheet on the other side was peeled off. The back curve side of the front lens member was contraposed with the back lens member in parallel and pressed thereto, and the two members were fixed.

### (5) Forming an antifogging film 6 on the back curve side of the front lens member 1 and the front curve side of the back lens member 2:

An ethyl alcohol solution for forming an antifogging film mainly containing a polyvinyl pyrolidone and a surfactant was injected into the air pressure adjusting hole 4 with 2mm diameter of the back lens member 2.

The double lens was shook evenly to allow the ethyl alcohol solution for forming an antifogging film to spread all over, and put in a centrifuge. And the remaining ethyl alcohol solution for forming an antifogging film was discharged through the air pressure adjusting hole 4 by a centrifugation method.

After that, the double lens was put in a decompression drying machine to allow the ethyl alcohol solution for forming an antifogging film to be dried out to form an antifogging film 6.

### (6) Waterproof of the air adjusting hole of the double lens and finishing of the double lens:

A fluorinated porous sheet of a diameter 4 mm having an adhesive ("Gore-Tex" manufactured by Japan Gore-Tex Inc.) was pasted over the air adjusting hole 4 of 2mm diameter formed on the back lens member 2, and the double lens was completed.

### (7) Preparation of a goggle frame:

The mold having a hole to fitly receive the double lens, two air holes of 2 cm² on the upper side of the goggle frame and two air holes of 2 cm² on the lower part of the goggle frame, and designed to be a cylindrical shape so as to easily fit with a user's face was used. With this mold, a polyurethane resin was injection-molded so as to prepare the goggle frame.

### (8) Waterproof of the air hole of the goggle frame:

An open-cellular polyurethane sheet of a thickness of 2 mm was pasted over each air hole by an adhesive.

### (9) Attachment of the goggle frame to a on-face member and finishing of the goggle frame:

An open-cellular polyurethane sheet of a thickness of 15 mm was cutting out into a shape with a width of 15 mm in accordance with a shape of a face contacting part of the goggle frame to form a ring-shaped on-face member. It was pasted on a periphery part of the goggle frame with an adhesive to finish the goggle frame.

### (10) Attachment of the double lens to the goggle frame:

The double lens part was inserted in a receiving hole of the goggle frame and fitted in a groove around the receiving hole so as to fix the double lens to the frame.

### (11) Preparation of a fitting belt to a user's head and finishing of the goggles:

A fitting belt to a user's head was made with a belt of elastic string with rubber sting wounded by synthetic fiber and a clip. The belt was attached to the goggle frame to complete goggles.

### (Antifogging Test)

Putting the above-described goggles on his face in at an ambient temperature, a wearer entered an environmental test room with a temperature of -5°C. Even after one hour had passed, no fogging was generated on any region of the double lens.

In addition, even if the double lens was twisted, no separation was found in any fixing part between the front lens member 1, the back lens member 2 and the gasket 3.

### [First Comparative Embodiment]

When forming an antifogging film on a back curve side of a front lens member, no masking sheet was used. And such a front lens member was dipped into a solution for forming an antifogging film and provided with the antifogging film both on the front curve side and the back curve side of the front lens member entirely. Except for the above, the same way as in the first embodiment was used and goggles were produced.

In the same way as in the first embodiment, the antifogging test was carried out at a temperature of -5°C, and even in one hour, no fogging was generated on any region of the double lens.

However, by twisting the double lens, the fixing part between the front lens member and the gasket was easily separated.

In addition, when the front curve side of the front lens member was wiped with a towel in order to remove an attached foreign substance, the antifogging film was damaged.

### [Second Comparative Embodiment]

Different from the second embodiment, no ethyl alcohol solution for forming an antifogging film was injected into the air pressure adjusting hole and no antifogging film was formed on the back curve side of the front lens member and the front curve side of the back lens member. Except for the above, the same way as in the second embodiment was used and goggles were produced.

Even if the prepared double lens was twisted, no separation of the fixing part on the gasket was found in any region of the double lens. However, the antifogging test at a temperature of -5°C according to the same method as the first embodiment proved that fogging was generated on the back curve side of the front lens in twenty minutes.

## Claims

1. A double lens including a front lens member, a back lens member and a gasket wherein a back curve side of the front lens member and a front curve side of the back lens member are contraposed in parallel, and fixed together with the gasket, at least one of the back curve side of the front lens member and the front curve side of the back lens member has an antifogging film which is formed avoiding part of a gasket bonding area, and the antifogging film is formed after the gasket is fixed on a side to be provided with the antifogging film or a masking member with an equivalent shape of the gasket is applied on the side.

2. The double lens according to Claim 1, wherein the antifogging film is formed by a printing method avoiding the equivalent shape of the gasket.

3. The double lens according to Claim 1, wherein the antifogging film is formed after the two lens members are fixed together with the gasket and an antifogging solution is inserted inside of the double lens through an air hole in the lens or the gasket.
